# EUROPEAN PATENT APPLICATION

(11) **EP 3 682 875 A2**
(43) Date of publication of application: **22.07.2020**
(21) Application number: 19217737.6
(22) Date of filing: 27.06.2013
(51) Int. Cl.: A61K 31/202, A61P 3/00

(54) **METHODS OF TREATING PEDIATRIC METABOLIC SYNDROME**

(30) Priority: 29.06.2012 US 201261666437 P
(62) Divisional of application: 13808719.2
(71) Applicant: Amarin Pharmaceuticals Ireland Limited, Dublin 2 (IE)
(72) Inventor: SONI, Paresh, Mystic, CT 06355 (US)
(74) Representative: Carridge, Andrew Edward

(57) **Abstract**

In various embodiments, the present invention provides methods of treating and/or preventing pediatric metabolic syndrome comprising administering to a subject in need thereof a pharmaceutical composition comprising eicosapentaenoic acid or a derivative thereof.

## Description

### PRIORITY CLAIM

This application claims priority to U.S. provisional patent application serial no. 61/666,437, filed June 29, 2012, the entire contents of which is incorporated herein by reference.

### BACKGROUND

Nearly 200 million children under the age of 17 are at risk of developing pediatric metabolic syndrome, a precursor of Type 2 diabetes and cardiovascular disease. It is estimated that 26 million people in the United States suffer from diabetes, and over 70 million people in the United States alone suffer from a cardiovascular disease or disorder including but not limited to high blood pressure, coronary heart disease, dyslipidemia, congestive heart failure and stroke. Treatment of metabolic syndrome-including pediatric metabolic syndrome-primarily involves changes in the subject's lifestyle. However, modifications to a subject's lifestyle are often labor-intensive and expensive; children and adolescents often fail to adhere to the prescribed regimens for a variety of reasons. Treatment of pediatric metabolic syndrome through surgery (e.g., malabsorptive procedures such as jejunoilial bypass, or restrictive procedures such as gastric banding) carry significant risks, including iron deficiency anemia, transient folate deficiency, cholecystectomy, bowel obstruction and incisional hernia. Weight-loss medications also carry significant side effect risks, especially in children and adolescents. Accordingly, only a few active agents are approved for use in children. One such agent, orlistat (ALLI®, XENICAL®) is only approved for patients age 12 or older and can cause a number of side effects, including oily or fatty stools, orange or brown colored oil in stool, loose stools, inability to control bowel movements, stomach pain, nausea, vomiting, diarrhea, rectal pain, weakness, dark urine, itching, jaundice, headache, back pain, and/or mild skin rash. A need exists for improved pharmaceutical-based treatments of pediatric metabolic syndrome.

### SUMMARY

In various embodiments, the present invention provides methods of treating and/or preventing pediatric metabolic syndrome comprising administering to a subject in need thereof a pharmaceutical composition comprising eicosapentaenoic acid or a derivative thereof. In one embodiment, the method comprises administering about 1 g to about 4 g of ethyl eicosapentaenoate per day. In one embodiment, the composition contains not more than 10%, by weight, docosahexaenoic acid or derivative thereof, substantially no docosahexaenoic acid or derivative thereof, or no docosahexaenoic acid or derivative thereof. In another embodiment, eicosapentaenoic acid ethyl ester comprises at least 96%, by weight, of all fatty acids present in the composition; the composition contains not more than 4%, by weight, of total fatty acids other than eicosapentaenoic acid ethyl ester; and/or the composition contains about 0.1% to about 0.6% of at least one fatty acid other than eicosapentaenoic acid ethyl ester and docosahexaenoic acid (or derivative thereof).

In one embodiment, a pharmaceutical composition useful in accordance with the invention comprises, consists of or consists essentially of at least 95% by weight ethyl eicosapentaenoate (EPA-E), about 0.2% to about 0.5% by weight ethyl octadecatetraenoate (ODTA-E), about 0.05% to about 0.25% by weight ethyl nonaecapentaenoate (NDPA-E), about 0.2% to about 0.45% by weight ethyl arachidonate (AA-E), about 0.3% to about 0.5% by weight ethyl eicosatetraenoate (ETA-E), and about 0.05% to about 0.32% ethyl heneicosapentaenoate (HPA-E). In another embodiment, the composition is present in a capsule shell. In another embodiment, the composition contains substantially no or no amount of docosahexaenoic acid (DHA) or derivative thereof such as ethyl-DHA (DHA-E).

In another embodiment, the invention provides a method of treating and/or preventing pediatric metabolic syndrome comprising administering a composition as described herein to a subject in need thereof one to about four times per day.

These and other embodiments of the present invention will be disclosed in further detail herein below.

### DETAILED DESCRIPTION

While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading may be combined with embodiments illustrated under any other heading.

The use of numerical values in the various quantitative values specified in this application, unless expressly indicated otherwise, are stated as approximations as though the minimum and maximum values within the stated ranges were both preceded by the word "about." Also, the disclosure of ranges is intended as a continuous range including every value between the minimum and maximum values recited as well as any ranges that can be formed by such values. Also disclosed herein are any and all ratios (and ranges of any such ratios) that can be formed by dividing a disclosed numeric value into any other disclosed numeric value. Accordingly, the skilled person will appreciate that many such ratios, ranges, and ranges of ratios can be unambiguously derived from the numerical values presented herein and in all instances such ratios, ranges, and ranges of ratios represent various embodiments of the present invention.

In one embodiment, the invention provides a method for treatment and/or prevention of pediatric metabolic syndrome. The term "pediatric metabolic syndrome" herein refers to: the condition commonly known as metabolic syndrome in a subject (e.g., a patient) of age 18 years or younger as defined by any reputable publication or organization, such as but by no means limited to: Cook et al., Arch. Pediatr. Adolesc. Med., vol. 157, pp. 821-827 (2003); de Ferranti et al., Circulation, vol. 110, pp. 2494-97 (2004); Cruz et al., J. Clin. Endocrinol. Metab., vol. 89, pp. 108-13 (2004); Weiss et al., N. Engl. J. Med., vol. 350, pp. 2362-74 (2004); Ford et al., Diabetes Care, vol. 28, pp. 878-81 (2005); Int'l Diabetes Federation, Metabolic Syndrome in Children and Adolescents, p. 10 (Oct. 2007); and/or Int'l Diabetes Federation, Metabolic Syndrome in Children and Adolescents, pp. 10-11 (2006). The term "pediatric metabolic syndrome" herein also refers to any symptom, sub-indication, attribute, characteristic, or other feature of the condition as a whole.

The term "treatment" in relation a given disease or disorder, includes, but is not limited to, inhibiting the disease or disorder, for example, arresting the development of the disease or disorder; relieving the disease or disorder, for example, causing regression of the disease or disorder; or relieving a condition caused by or resulting from the disease or disorder, for example, relieving, preventing or treating symptoms of the disease or disorder. The term "prevention" in relation to a given disease or disorder means: preventing the onset of disease development if none had occurred, preventing the disease or disorder from occurring in a subject that may be predisposed to the disorder or disease but has not yet been diagnosed as having the disorder or disease, and/or preventing further disease/disorder development if already present.

In one embodiment, the present invention provides a method of treating pediatric metabolic syndrome in a subject in need thereof, the method comprising identifying a subject as having pediatric metabolic syndrome, and administering to the subject about 1 g to about 4 g of ethyl eicosapentaenoate per day. In some embodiments, the composition contains substantially no DHA or derivative thereof. In some embodiments, the subject has one or more of: excessive belly fat, hypertension, hyperlipidemia, and/or hyperglycemia.

In some embodiments, the subject has three or more of: (a) a fasting glucose level of at least 110 mg/dL; (b) a waist circumference value in at least the 90th percentile based on an age and a gender associated with the subject; (c) a triglyceride level of at least 110 mg/dL; (d) an HDL-C level of no more than about 40 mg/dL; and (e) a blood pressure level in at least the 90th percentile based on the age, gender and height associated with the subject. In some embodiments, the subject has four or more of the above. In some embodiments, the subject has all five of the above.

In some embodiments, the subject has three or more of: (a) a fasting glucose level of at least 110 mg/dL; (b) a waist circumference value in at least the 75th percentile; (c) a triglyceride level of at least 100 mg/dL; (d) an HDL-C level of no more than about 50 mg/dL; and (e) a blood pressure level in at least the 90th percentile. In some embodiments, the subject has four or more of the above. In some embodiments, the subject has all five of the above.

In some embodiments, the subject has three or more of: (a) an impaired glucose tolerance as determined by having two-hour glucose levels of 140 mg/dL to 199 mg/dL on the 75-gram oral glucose tolerance test; (b) a waist circumference value in at least the 90th percentile based on an age, a gender, and a race associated with the subject; (c) a triglyceride level in at least the 90th percentile based on the age and gender associated with the subject; (d) an HDL-C level in no more than the 10th percentile based on the age and gender associated with the subject; and (e) a blood pressure level in at least the 90th percentile based on the age, the gender and a height associated with the subject. In some embodiments, the subject has four or more of the above. In some embodiments, the subject has all five of the above.

In some embodiments, the subject has three or more of: (a) an impaired glucose tolerance as determined by having two-hour glucose levels of 140 mg/dL to 199 mg/dL on the 75-gram oral glucose tolerance test; (b) a body mass index ("BMI-Z") score of at least 2.0 based on an age and a gender associated with the subject; (c) a triglyceride level in at least the 95th percentile based on the age, the gender and a race associated with the subject; (d) an HDL-C level in no more than the 5th percentile based on the age, the gender, and the race associated with the subject; and (e) a blood pressure level in at least the 95th percentile based on the age, gender and height associated with the subject. In some embodiments, the subject has four or more of the above. In some embodiments, the subject has all five of the above.

In some embodiments, the subject has three or more of: (a) a fasting glucose level of at least 110 mg/dL; (b) a waist circumference value in at least the 90th percentile based on a gender associated with the subject; (c) a triglyceride level of at least 110 mg/dL based on the age associated with the subject; (d) an HDL-C level of no more than 40 mg/dL; and (e) a blood pressure level in at least the 90th percentile based on the age, the gender and a height associated with the subject. In some embodiments, the subject has four or more of the above. In some embodiments, the subject has all five of the above.

In some embodiments, the subject has pediatric metabolic syndrome as defined by the International Diabetes Federation (October 2007). For example, in some embodiments, the subject is at least 6 years old and less than 10 years old, and has a waist circumference value in at least the 90th percentile. In other embodiments, the subject is at least 10 years old and less than 16 years old, has a waist circumference value in at least the 90th percentile, and further has at least two of: (a) a triglyceride level of at least 150 mg/dL; (b) an HDL-C level of no more than 40 mg/dL; (c) a blood pressure of at least 130 mmHg systolic or at least 85 mmHg diastolic; and (d) a blood glucose level of at least 100 mg/dL. In still other embodiments, the subject is at least 16 years old and: (a) a body mass index of at least 30 kg/m² (or a waist measurement of: at least 102 cm if the subject is male and lives in North America or at least 88 cm if the subject is female and lives in North America, at least 94 cm if the subject is male, does not live in North America, and has an ethnicity associated with Europe, sub-Saharan Africa, Eastern Mediterranean or the Middle East, or at least 80 cm if the subject is female, does not live in North America, and has an ethnicity associated with Europe, sub-Saharan Africa, Eastern Mediterranean or the Middle East, at least 90 cm if the subject is male, does not live in North America, and has an ethnicity associated with South Asia, China, Japan, South America or Central America, or at least 80 cm if the subject is female, does not live in North America, and has an ethnicity associated with South Asia, China, Japan, South America or Central America); and (b) at least two of: (i) a triglyceride level of at least 150 mg/dL, (ii) an HDL-C level of no more than 40 mg/dL if the subject is male or of no more than 50 mg/dL if the subject is female, (iii) a blood pressure of at least 130 mmHg systolic or at least 85 mmHg diastolic; and (iv) a fasting plasma glucose level of at least 100 mg/dL.

In some embodiments, the method of the pharmaceutical composition is administered to the subject 1 to 4 times per day. In some embodiments, the pharmaceutical composition is present in a capsule, such as a gelatin capsule. In some embodiments, the capsule is coated with a coating, such as an enteric coating. In some embodiments, the ethyl eicosapentaenoate is administered in two or more divided doses per day, for example in two, three, four, five, six, seven, eight, or more than eight divided doses per day.

In some embodiments, the ethyl eicosapentaenoate represents at least about 80%, at least about 90%, at least about 95%, at least about 96%, or greater than 96%, by weight, of all fatty acids administered to the subject. In some embodiments, docosahexaenoic acid and its derivatives (e.g., an ester of docosahexaenoic acid such as ethyl docosahexaenoate) represent no more than about 10%, no more than about 9%, no more than about 8%, no more than about 7%, no more than about 6%, no more than about 5%, no more than about 4%, no more than about 3%, or no more than about 2%, by weight, of all fatty acids administered to the subject.

In some embodiments, the subject is on concomitant lipid-altering therapy. For example, the subject may be taking a statin, ezetimibe, or any other fatty acid composition (*e.g.*, Lovaza or a fatty acid dietary supplement). In some embodiments, the subject does ingests more than 200 mg of niacin per day. In some embodiments, the subject ingests more than about 1 g (*e.g.,* about 1 g, about 2 g, about 3 g, about 4 g, about 5 g, or more than about 5 g) of fatty acid compounds per day, other than the pharmaceutical composition disclosed herein.

In some embodiments, the subject is not on concomitant lipid-altering therapy. For example, the subject may not be taking a statin, ezetimibe, or any other fatty acid composition (*e.g.*, Lovaza or a fatty acid dietary supplement). In some embodiments, the subject does not ingest more than 200 mg of niacin per day. In some embodiments, the subject does not ingest more than about 1 g (*e.g.,* less than 1 g, less than 0.9 g, less than 0.8 g, less than 0.7 g, less than 0.6 g, less than 0.5 g, less than 0.4 g, less than 0.3 g, less than 0.2 g, or less than 0.1 g) of fatty acid compounds per day, other than the pharmaceutical composition disclosed herein.

In some embodiments, the present invention provides a method of treating pediatric metabolic syndrome in a subject in need thereof, the method comprising identifying a subject as having pediatric metabolic syndrome, and administering to the subject about 1 g to about 4 g of a pharmaceutical composition comprising at least about 96%, by weight, ethyl eicosapentaenoate, wherein the composition contains substantially no DHA or derivative thereof, and wherein the method further comprises a step of measuring a baseline lipid profile in the subject prior to administering the pharmaceutical composition to said subject. In some embodiments, the baseline lipid profile comprises one or more of: a fasting baseline triglyceride level, a baseline non-HDL-C value, a baseline total cholesterol value, a baseline VLDL-C level, and/or a baseline HDL-C value. In some embodiments, the subject has one or more of: a fasting baseline triglyceride level of about 135 mg/dL to about 1500 mg/dL, a baseline non-HDL-C value of about 200 mg/dL to about 300 mg/dL; a baseline total cholesterol value of about 250 mg/dL to about 300 mg/dL; a baseline VLDL-C value of about 140 mg/dL to about 200 mg/dL; and/or a baseline HDL-C value of about 10 to about 80 mg/dL.

In some embodiments, the present invention provides a method of treating pediatric metabolic syndrome in a subject in need thereof, the method comprising identifying a subject as having pediatric metabolic syndrome, and administering to the subject about 1 g to about 4 g of a pharmaceutical composition comprising at least about 96%, by weight, ethyl eicosapentaenoate, wherein the composition contains substantially no DHA or derivative thereof, wherein after administering the pharmaceutical composition to the subject daily for about 12 weeks, the subject exhibits one or more of: (a) reduced triglyceride levels compared to baseline; (b) reduced Apo B levels compared to baseline; (c) increased HDL-C levels compared to baseline; (d) a reduction in non-HDL-C levels compared to baseline; and/or (e) a reduction in VLDL levels compared to baseline.

In some embodiments, the present invention provides a method of treating pediatric metabolic syndrome in a subject in need thereof, the method comprising identifying a subject as having pediatric metabolic syndrome, and administering to the subject about 1 g to about 4 g of a pharmaceutical composition comprising at least about 96%, by weight, ethyl eicosapentaenoate, wherein the composition contains substantially no DHA or derivative thereof, wherein after administering the pharmaceutical composition to the subject daily for about 12 weeks, the subject exhibits one or more of:
(a) a reduction in triglyceride level of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(b) a less than 30% increase, less than 20% increase, less than 10% increase, less than 5% increase or no increase in non-HDL-C levels or a reduction in non-HDL-C levels of at least about 1%, at least about 3%, at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(c) substantially no change in HDL-C levels, no change in HDL-C levels, or an increase in HDL-C levels of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline; and/or
(d) a less than 60% increase, a less than 50% increase, a less than 40% increase, a less than 30% increase, less than 20% increase, less than 10% increase, less than 5% increase or no increase in LDL-C levels or a reduction in LDL-C levels of at least about 5%, at least about 10%, at least about 15%, at least about 20%, at least about 25%, at least about 30%, at least about 35%, at least about 40%, at least about 45%, at least about 50%, at least about 55%, at least about 55% or at least about 75% (actual % change or median % change) as compared to baseline.

In some embodiments, the present invention provides a method of treating pediatric metabolic syndrome in a subject in need thereof, the method comprising identifying a subject as having pediatric metabolic syndrome, and administering to the subject about 1 g to about 4 g of a pharmaceutical composition comprising at least about 96%, by weight, ethyl eicosapentaenoate, wherein the composition contains substantially no DHA or derivative thereof, wherein after administering the pharmaceutical composition to the subject daily for about 12 weeks, the subject exhibits one or more of:
(a) a reduction in triglyceride level of at least about 30%, at least about 35%, at least about 40%, at least about 45%, at least about 50%, at least about 55% or at least about 75% (actual % change or median % change) as compared to baseline;
(b) no increase in non-HDL-C levels as compared to baseline;
(c) no decrease in HDL-C levels compared to baseline; and/or
(d) a less than 30% increase, less than 20% increase, less than 10% increase, less than 5% increase or no increase in LDL-C levels or a reduction in LDL-C levels of at least about 5%, at least about 10%, at least about 15%, at least about 20%, at least about 25%, at least about 30%, at least about 35%, at least about 40%, at least about 45%, at least about 50%, at least about 55%, at least about 55% or at least about 75% (actual % change or median % change) as compared to baseline.

In some embodiments, the present invention provides a method of treating pediatric metabolic syndrome in a subject in need thereof, the method comprising identifying a subject as having pediatric metabolic syndrome, and administering to the subject about 1 g to about 4 g of a pharmaceutical composition comprising at least about 96%, by weight, ethyl eicosapentaenoate, wherein the composition contains substantially no DHA or derivative thereof, wherein after administering the pharmaceutical composition to the subject daily for about 12 weeks, the subject exhibits one or more of the following outcomes:
(a) reduced triglyceride levels compared to baseline;
(b) reduced Apo B levels compared to baseline;
(c) increased HDL-C levels compared to baseline;
(d) no increase in LDL-C levels compared to baseline;
(e) a reduction in LDL-C levels compared to baseline;
(f) a reduction in non-HDL-C levels compared to baseline;
(g) a reduction in VLDL levels compared to baseline;
(h) an increase in apo A-I levels compared to baseline;
(i) an increase in apo A-I/apo B ratio compared to baseline;
(j) a reduction in lipoprotein a levels compared to baseline;
(k) a reduction in LDL particle number compared to baseline;
(l) an increase in LDL size compared to baseline;
(m) a reduction in remnant-like particle cholesterol compared to baseline;
(n) a reduction in oxidized LDL compared to baseline;
(o) a less than 5% change in fasting plasma glucose (FPG) compared to baseline;
(p) a less than 5% change in hemoglobin A_{1c} (HbA_{1c}) compared to baseline;
(q) a reduction in homeostasis model insulin resistance compared to baseline;
(r) a reduction in lipoprotein associated phospholipase A2 compared to baseline;
(s) a reduction in intracellular adhesion molecule compared to baseline;
(t) a reduction in interleukin-6 compared to baseline;
(u) a reduction in plasminogen activator inhibitor compared to baseline;
(v) a reduction in high sensitivity C-reactive protein (hsCRP) compared to baseline;
(w) an increase in serum phospholipid EPA compared to baseline; and/or
(x) an increase in red blood cell membrane EPA compared to baseline.

In one embodiment, methods of the present invention comprise measuring baseline levels of one or more markers set forth in (a) - (x) above prior to dosing the subject or subject group. In another embodiment, the methods comprise administering a composition as disclosed herein to the subject after baseline levels of one or more markers set forth in (a) - (x) are determined, and subsequently taking an additional measurement of said one or more markers.

In another embodiment, upon treatment with a composition of the present invention, for example over a period of about 1 to about 200 weeks, about 1 to about 100 weeks, about 1 to about 80 weeks, about 1 to about 50 weeks, about 1 to about 40 weeks, about 1 to about 20 weeks, about 1 to about 15 weeks, about 1 to about 12 weeks, about 1 to about 10 weeks, about 1 to about 5 weeks, about 1 to about 2 weeks or about 1 week, the subject or subject group exhibits any 2 or more of, any 3 or more of, any 4 or more of, any 5 or more of, any 6 or more of, any 7 or more of, any 8 or more of, any 9 or more of, any 10 or more of, any 11 or more of, any 12 or more of, any 13 or more of, any 14 or more of, any 15 or more of, any 16 or more of, any 17 or more of, any 18 or more of, any 19 or more of, any 20 or more of, any 21 or more of, any 22 or more of, any 23 or more, or all 24 of outcomes (a) - (x) described immediately above.

Parameters (a) - (x) can be measured in accordance with any clinically acceptable methodology. For example, triglycerides, total cholesterol, HDL-C and fasting blood sugar can be sample from serum and analyzed using standard photometry techniques. VLDL-TG, LDL-C and VLDL-C can be calculated or determined using serum lipoprotein fractionation by preparative ultracentrifugation and subsequent quantitative analysis by refractometry or by analytic ultracentrifugal methodology. Apo A1, Apo B and hsCRP can be determined from serum using standard nephelometry techniques. Lipoprotein (a) can be determined from serum using standard turbidimetric immunoassay techniques. LDL particle number and particle size can be determined using nuclear magnetic resonance (NMR) spectrometry. Remnants lipoproteins and LDL-phospholipase A2 can be determined from EDTA plasma or serum and serum, respectively, using enzymatic immunoseparation techniques. Oxidized LDL, intercellular adhesion molecule-1 and interleukin-2 levels can be determined from serum using standard enzyme immunoassay techniques. These techniques are described in detail in standard textbooks, for example Tietz Fundamentals of Clinical Chemistry, 6th Ed. (Burtis, Ashwood and Borter Eds.), WB Saunders Company.

In a related embodiment, the reductions or increases of parameters (a) - (x) above are statistically significant.

In another embodiment, upon treatment with a composition of the present invention, the subject or subject group exhibits one or more of the following outcomes:
(a) a reduction in triglyceride level of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(b) a less than 30% increase, less than 20% increase, less than 10% increase, less than 5% increase or no increase in non-HDL-C levels or a reduction in non-HDL-C levels of at least about 1%, at least about 3%, at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(c) substantially no change in HDL-C levels, no change in HDL-C levels, or an increase in HDL-C levels of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(d) a less than 60% increase, a less than 50% increase, a less than 40% increase, a less than 30% increase, less than 20% increase, less than 10% increase, less than 5% increase or no increase in LDL-C levels or a reduction in LDL-C levels of at least about 5%, at least about 10%, at least about 15%, at least about 20%, at least about 25%, at least about 30%, at least about 35%, at least about 40%, at least about 45%, at least about 50%, at least about 55%, at least about 55% or at least about 75% (actual % change or median % change) as compared to baseline;
(e) a decrease in Apo B levels of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) as compared to baseline;
(f) a reduction in VLDL levels of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(g) an increase in apo A-I levels of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(h) an increase in apo A-I/apo B ratio of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(i) a reduction in lipoprotein (a) levels of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(j) a reduction in mean LDL particle number of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(k) an increase in mean LDL particle size of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(l) a reduction in remnant-like particle cholesterol of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(m) a reduction in oxidized LDL of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(n) substantially no change, no significant change, or a reduction (e.g. in the case of a diabetic subject) in fasting plasma glucose (FPG) of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(o) substantially no change, no significant change or a reduction in hemoglobin A_{1c} (HbA_{1c}) of at least about 5%, at least about 10%, at least about 15%, at least about 20%, at least about 25%, at least about 30%, at least about 35%, at least about 40%, at least about 45%, or at least about 50% (actual % change or median % change) compared to baseline;
(p) a reduction in homeostasis model index insulin resistance of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(q) a reduction in lipoprotein associated phospholipase A2 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(r) a reduction in intracellular adhesion molecule-1 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(s) a reduction in interleukin-6 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(t) a reduction in plasminogen activator inhibitor-1 of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(u) a reduction in high sensitivity C-reactive protein (hsCRP) of at least about 5%, at least about 10%, at least about 15%, at least about 20%, at least about 25%, at least about 30%, at least about 35%, at least about 40%, at least about 45%, at least about 50%, or at least about 100% (actual % change or median % change) compared to baseline;
(v) an increase in serum, plasma and/or RBC EPA of at least about 5%, at least about 10%, at least about 15%, at least about 20%, at least about 25%, at least about 30%, at least about 35%, at least about 40%, at least about 45%, at least about 50%, at least about 100%, at least about 200% or at least about 400% (actual % change or median % change) compared to baseline;
(w) an increase in serum phospholipid and/or red blood cell membrane EPA of at least about 5%, at least about 10%, at least about 15%, at least about 20%, at least about 25%, at least about 30%, at least about 35%, at least about 40%, at least about 45%, r at least about 50%, at least about 100%, at least about 200%, or at least about 400% (actual % change or median % change) compared to baseline;
(x) a reduction or increase in one or more of serum phospholipid and/or red blood cell DHA, DPA, AA, PA and/or OA of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) compared to baseline; and/or
(y) a reduction in total cholesterol of at least about 5%, at least about 10%, at least about 15%, at least about 20%, at least about 25%, at least about 30%, at least about 35%, at least about 40%, at least about 45%, at least about 50%, at least about 55% or at least about 75% (actual % change or median % change) compared to baseline.

In one embodiment, methods of the present invention comprise measuring baseline levels of one or more markers set forth in (a) - (y) prior to dosing the subject or subject group. In another embodiment, the methods comprise administering a composition as disclosed herein to the subject after baseline levels of one or more markers set forth in (a) - (y) are determined, and subsequently taking a second measurement of the one or more markers as measured at baseline for comparison thereto.

In another embodiment, upon treatment with a composition of the present invention, for example over a period of about 1 to about 200 weeks, about 1 to about 100 weeks, about 1 to about 80 weeks, about 1 to about 50 weeks, about 1 to about 40 weeks, about 1 to about 20 weeks, about 1 to about 15 weeks, about 1 to about 12 weeks, about 1 to about 10 weeks, about 1 to about 5 weeks, about 1 to about 2 weeks or about 1 week, the subject or subject group exhibits any 2 or more of, any 3 or more of, any 4 or more of, any 5 or more of, any 6 or more of, any 7 or more of, any 8 or more of, any 9 or more of, any 10 or more of, any 11 or more of, any 12 or more of, any 13 or more of, any 14 or more of, any 15 or more of, any 16 or more of, any 17 or more of, any 18 or more of, any 19 or more of, any 20 or more of, any 21 or more of, any 22 or more of, any 23 or more of, any 24 or more of, or all 25 or more of outcomes (a) - (y) described immediately above.

Parameters (a) - (y) can be measured in accordance with any clinically acceptable methodology. For example, triglycerides, total cholesterol, HDL-C and fasting blood sugar can be sample from serum and analyzed using standard photometry techniques. VLDL-TG, LDL-C and VLDL-C can be calculated or determined using serum lipoprotein fractionation by preparative ultracentrifugation and subsequent quantitative analysis by refractometry or by analytic ultracentrifugal methodology. Apo A1, Apo B and hsCRP can be determined from serum using standard nephelometry techniques. Lipoprotein (a) can be determined from serum using standard turbidimetric immunoassay techniques. LDL particle number and particle size can be determined using nuclear magnetic resonance (NMR) spectrometry. Remnants lipoproteins and LDL-phospholipase A2 can be determined from EDTA plasma or serum and serum, respectively, using enzymatic immunoseparation techniques. Oxidized LDL, intercellular adhesion molecule-1 and interleukin-6 levels can be determined from serum using standard enzyme immunoassay techniques. These techniques are described in detail in standard textbooks, for example Tietz Fundamentals of Clinical Chemistry, 6th Ed. (Burtis, Ashwood and Borter Eds.), WB Saunders Company.

In one embodiment, subjects fast for up to 12 hours prior to blood sample collection, for example about 10 hours.

In some embodiments, the subject is pre-diabetic. For example and without limitation, a subject may be considered to be pre-diabetic for purposes of this disclosure if he or she exhibits one or more of: darkened areas of skin (*i.e.,* acanthosis nigricans), increased thirst, frequent urination, fatigue and/or blurred vision.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) a second active agent selected from the group consisting of: gastric and pancreatic lipase inhibitors, serotonin and adrenaline reuptake inhibitors, appetite suppressants, biguanides, norandrenergic drugs, pancreatic insulin suppressants, anticonvulsants, endocannabinoid type 1 receptor blockers, and combinations thereof. In various embodiments, the pharmaceutical composition is co-administered or administered concomitantly with the second active agent. In some embodiments, the second active agent is administered at a dose that is less than a dose that would ordinarily be prescribed to the subject if the second active agent was prescribed without concomitant administration of the pharmaceutical composition comprising EPA. The terms "co-administered," "concomitant administration," and "administered concomitantly" are used interchangeably herein and each refer to, for example, administration of two or more agents (e.g., EPA or a derivative thereof and a second active agent) at the same time, in the same dosage unit, one immediately after the other, within five minutes of each other, within ten minutes of each other, within fifteen minutes of each other, within thirty minutes of each other, within one hour of each other, within two hours of each other, within four hours of each other, within six hours of each other, within twelve hours of each other, within one day of each other, within one week of each other, within two weeks of each other, within one month of each other, within two months of each other, within six months of each other, within one year of each other, *etc.*

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) a gastric and pancreatic lipase inhibitor. In some embodiments, the gastric and pancreatic lipase inhibitor comprises orlistat. In various embodiments, the pharmaceutical composition is co-administered or administered concomitantly with the gastric and pancreatic lipase inhibitor (*e.g.*, orlistat). In some embodiments, the gastric and pancreatic lipase inhibitor is administered at a dose that is less than a dose that would ordinarily be prescribed to the subject if the gastric and pancreatic lipase inhibitor was prescribed without concomitant administration of the pharmaceutical composition comprising EPA. For example and without limitation, in an embodiment wherein the gastric and pancreatic lipase inhibitor is orlistat, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 360 mg per day or less of orlistat, for example about 360 mg per day, about 350 mg per day, about 340 mg per day, about 330 mg per day, about 320 mg per day, about 310 mg per day, about 300 mg per day, about 290 mg per day, about 280 mg per day, about 270 mg per day, about 260 mg per day, about 250 mg per day, about 240 mg per day, about 230 mg per day, about 220 mg per day, about 210 mg per day, about 200 mg per day, about 190 mg per day, about 180 mg per day, about 170 mg per day, about 160 mg per day, about 150 mg per day, about 140 mg per day, about 130 mg per day, about 120 mg per day, about 110 mg per day, about 100 mg per day, about 90 mg per day, about 80 mg per day, about 70 mg per day, about 60 mg per day, about 50 mg per day, about 40 mg per day, about 30 mg per day, about 20 mg per day, or about 10 mg per day of orlistat.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) a serotonin and adrenaline reuptake inhibitor. In some embodiments, the serotonin and adrenaline reuptake inhibitor comprises sibutramine. In various embodiments, the pharmaceutical composition is co-administered or administered concomitantly with the serotonin and adrenaline reuptake inhibitor (*e.g*., sibutramine). In some embodiments, the serotonin and adrenaline reuptake inhibitor is administered at a dose that is less than a dose that would ordinarily be prescribed to the subject if the serotonin and adrenaline reuptake inhibitor was prescribed without concomitant administration of the pharmaceutical composition comprising EPA. For example and without limitation, in an embodiment wherein the serotonin and adrenaline reuptake inhibitor is suibutramine, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 15 mg per day or less of sibutramine, for example about 15 mg per day, about 14 mg per day, about 13 mg per day, about 12 mg per day, about 11 mg per day, about 10 mg per day, about 9 mg per day, about 8 mg per day, about 7 mg per day, about 6 mg per day, about 5 mg per day, about 4 mg per day, about 3 mg per day, about 2 mg per day, or about 1 mg per day of sibutramine.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) a biguanide. In some embodiments, the biguanide comprises metformin. In various embodiments, the pharmaceutical composition is co-administered or administered concomitantly with the biguanide (*e.g.*, metformin). In some embodiments, the biguanide is administered at a dose that is less than a dose that would ordinarily be prescribed to the subject if the biguanide was prescribed without concomitant administration of the pharmaceutical composition comprising EPA. For example and without limitation, in an embodiment wherein the biguanide is metformin, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 2,000 mg per day or less of metformin, for example about 2,000 mg per day, about 1,900 mg per day, about 1,800 mg per day, about 1,700 mg per day, about 1,600 mg per day, about 1,500 mg per day, about 1,400 mg per day, about 1,300 mg per day, about 1,200 mg per day, about 1,100 mg per day, about 1,000 mg per day, about 900 mg per day, about 800 mg per day, about 700 mg per day, about 600 mg per day, about 500 mg per day, about 400 mg per day, about 300 mg per day, about 200 mg per day, or about 100 mg per day of metformin.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) a norandrenergic drug. In some embodiments, the norandrenergic drug comprises phentermine, chlorpehntermine, phenylpropanolamine, amphepramone, and/or combinations thereof. In various embodiments, the pharmaceutical composition is co-administered or administered concomitantly with the norandrenergic drug(s). In some embodiments, the norandrenergic drug is administered at a dose that is less than a dose that would ordinarily be prescribed to the subject if the norandrenergic drug was prescribed without concomitant administration of the pharmaceutical composition comprising EPA. For example and without limitation, in an embodiment wherein the norandrenergic drug is metformin, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 37.5 mg per day or less of phentermine, for example about 37.5 mg per day, 35 mg per day, about 32.5 mg per day, about 30 mg per day, about 27.5 mg per day, about 25 mg per day, about 22.5 mg per day, about 20 mg per day, about 17.5 mg per day, about 15 mg per day, about 12.5 mg per day, about 10 mg per day, about 7.5 mg per day, about 5 mg per day, or about 2.5 mg per day of phentermine.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) a pancreatic insulin suppressant. In some embodiments, the pancreatic insulin suppressant comprises octreotide. In various embodiments, the pharmaceutical composition is co-administered or administered concomitantly with the pancreatic insulin suppressant (*e.g.*, octreotide). In some embodiments, the pancreatic insulin suppressant is administered at a dose that is less than a dose that would ordinarily be prescribed to the subject if the pancreatic insulin suppressant was prescribed without concomitant administration of the pharmaceutical composition comprising EPA. For example and without limitation, in an embodiment wherein the pancreatic insulin suppressant is octreotide, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 600 µg per day or less of octreotide, for example about 600 mg per day, 580 µg per day, about 560 µg per day, about 540 µg per day, about 520 µg per day, about 500 µg per day, about 480 µg per day, about 460 µg per day, about 440 µg per day, about 420 µg per day, about 400 µg per day, about 380 µg per day, about 360 µg per day, about 340 µg per day, about 320 µg per day, about 300 µg per day, about 280 µg per day, about 260 µg per day, about 240 µg per day, about 220 µg per day, about 200 µg per day, about 180 µg per day, about 160 µg per day, about 140 µg per day, about 120 µg per day, about 100 µg per day, about 80 µg per day, about 60 µg per day, about 40 µg per day, or about 20 µg per day of octreotide.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) an anticonvulsant. In some embodiments, the anticonvulsant comprises topiramate. In various embodiments, the pharmaceutical composition is co-administered or administered concomitantly with the anticonvulsant (*e.g.*, topiramate). In some embodiments, the anticonvulsant is administered at a dose that is less than a dose that would ordinarily be prescribed to the subject if the anticonvulsant was prescribed without concomitant administration of the pharmaceutical composition comprising EPA. For example and without limitation, in an embodiment wherein the anticonvulsant is topiramate, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 9 mg/kg per day or less of topiramate, for example about 9 mg/kg per day, about 8 mg/kg per day, about 7 mg/kg per day, about 6 mg/kg per day, about 5 mg/kg per day, about 4 mg/kg per day, about 3 mg/kg per day, about 2 mg/kg per day, about 1 mg/kg per day, about 0.9 mg/kg per day, about 0.8 mg/kg per day, about 0.7 mg/kg per day, about 0.6 mg/kg per day, about 0.5 mg/kg per day, about 0.4 mg/kg per day, about 0.3 mg/kg per day, about 0.2 mg/kg per day, or about 0.1 mg/kg per day of topiramate.

In some embodiments, the present invention provides a method of treating pediatric metabolic syndrome in a subject in need thereof, the method comprising identifying a subject as having pediatric metabolic syndrome, and administering to the subject (a) about 1 g to about 4 g of a pharmaceutical composition comprising at least about 96%, by weight, ethyl eicosapentaenoate, wherein the composition contains substantially no DHA or derivative thereof, and (b) an endocannabinoid type 1 (CB₁) receptor blocker. In some embodiments, the endocannabinoid type 1 receptor blocker comprises rimonabant and/or TM38837. In various embodiments, the pharmaceutical composition is co-administered or administered concomitantly with the endocannabinoid type 1 receptor blocker (*e.g.*, rimonabant and/or TM38837). In some embodiments, the endocannabinoid type 1 receptor blocker is administered at a dose that is less than a dose that would ordinarily be prescribed to the subject if the endocannabinoid type 1 receptor blocker was prescribed without concomitant administration of the pharmaceutical composition comprising EPA. For example and without limitation, in an embodiment wherein the endocannabinoid type 1 receptor blocker is rimonbant, the method comprises administering to the subject (a) about 1 g to about 4 g of a pharmaceutical composition comprising at least 96%, by weight, ethyl eicosapentaenoate, wherein the composition contains substantially no DHA or derivative thereof, and (b) about 20 mg per day or less of rimonabant, for example about 20 mg per day, about 19 mg per day, about 18 mg per day, about 17 mg per day, about 16 mg per day, about 15 mg per day, about 14 mg per day, about 13 mg per day, about 12 mg per day, about 11 mg per day, about 10 mg per day, about 9 mg per day, about 8 mg per day, about 7 mg per day, about 6 mg per day, about 5 mg per day, about 4 mg per day, about 3 mg per day, about 2 mg per day, about 1 mg per day of rimonabant.

In some embodiments, the pharmaceutical composition is packaged in blister packages of about 1 to about 20 capsules per sheet, for example 1 capsule per sheet, 2 capsules per sheet, 3 capsules per sheet, 4 capsules per sheet, 5 capsules per sheet, 6 capsules per sheet, 7 capsules per sheet, 8 capsules per sheet, 9 capsules per sheet, 10 capsules per sheet, 11 capsules per sheet, 12 capsules per sheet, 13 capsules per sheet, 14 capsules per sheet, 15 capsules per sheet, 16 capsules per sheet, 17 capsules per sheet, 18 capsules per sheet, 19 capsules per sheet, or 20 capsules per sheet.

In some embodiments, the pharmaceutical composition is packaged together with instructions for using the composition to treat pediatric metabolic syndrome. In some embodiments, the instructions include information on calculating a suitable daily dose for a subject. In some embodiments, the instructions include information for administering about 1 g to about 4 g daily of the pharmaceutical composition. In some embodiments, the instructions include information about certain drugs and/or dietary supplements to avoid including, for example, other omega-3 fatty acid pharmaceutical compositions such as Lovaza, omega-3 fatty acid containing dietary supplements, and/or food with high levels of omega-3 fatty acids. In some embodiments, the instructions include information for taking the pharmaceutical composition along with (*e.g.*, a co-administration) one or more additional active agents. In some embodiments, the additional active agent(s) is/are selected from the group consisting of: gastric and pancreatic lipase inhibitors, serotonin and adrenaline reuptake inhibitors, appetite suppressants, biguanides, norandrenergic drugs, pancreatic insulin suppressants, anticonvulsants, endocannabinoid type 1 receptor blockers, and combinations thereof.

In another embodiment, the subject or subject group being treated has a baseline triglyceride level (or median baseline triglyceride level in the case of a subject group), fed or fasting, of at least about 100 mg/dL, at least about 125 mg/dL, at least about 135 mg/dL, at least about 150 mg/dL, about 175 mg/dL, about 200 mg/dL, about 225 mg/dL, about 250 mg/dL, about 275 mg/dL, about 300 mg/dL, at least about 400 mg/dL, at least about 500 mg/dL, at least about 600 mg/dL, at least about 700 mg/dL, at least about 800 mg/dL, at least about 900 mg/dL, at least about 1000 mg/dL, at least about 1100 mg/dL, at least about 1200 mg/dL, at least about 1300 mg/dL, at least about 1400 mg/dL, or at least about 1500 mg/dL, for example about 400 mg/dL to about 2500 mg/dL, about 450 mg/dL to about 2000 mg/dL or about 500 mg/dL to about 1500 mg/dL.

In one embodiment, the subject or subject group being treated in accordance with methods of the invention has previously been treated with Lovaza® and has experienced an increase in, or no decrease in, LDL-C levels and/or non-HDL-C levels. In one such embodiment, Lovaza® therapy is discontinued and replaced by a method of the present invention.

In another embodiment, the subject or subject group being treated in accordance with methods of the invention exhibits a fasting baseline absolute plasma level of free EPA (or mean thereof in the case of a subject group) not greater than about 0.70 nmol/ml, not greater than about 0.65 nmol/ml, not greater than about 0.60 nmol/ml, not greater than about 0.55 nmol/ml, not greater than about 0.50 nmol/ml, not greater than about 0.45 nmol/ml, or not greater than about 0.40 nmol/ml. In another embodiment, the subject or subject group being treated in accordance with methods of the invention exhibits a baseline fasting plasma level (or mean thereof) of free EPA, expressed as a percentage of total free fatty acid, of not more than about 3%, not more than about 2.5%, not more than about 2%, not more than about 1.5%, not more than about 1%, not more than about 0.75%, not more than about 0.5%, not more than about 0.25%, not more than about 0.2% or not more than about 0.15%. In one such embodiment, free plasma EPA and/or total fatty acid levels are determined prior to initiating therapy.

In another embodiment, the subject or subject group being treated in accordance with methods of the invention exhibits a fasting baseline absolute plasma level of total fatty acid (or mean thereof) not greater than about 250 nmol/ml, not greater than about 200 nmol/ml, not greater than about 150 nmol/ml, not greater than about 100 nmol/ml, or not greater than about 50 nmol/ml.

In another embodiment, the subject or subject group being treated in accordance with methods of the invention exhibits a fasting baseline plasma, serum or red blood cell membrane EPA level not greater than about 70 µg/ml, not greater than about 60 µg/ml, not greater than about 50 µg/ml, not greater than about 40 µg/ml, not greater than about 30 µg/ml, or not greater than about 25 µg/ml.

In another embodiment, methods of the present invention comprise a step of measuring the subject's (or subject group's mean) baseline lipid profile prior to initiating therapy. In another embodiment, methods of the invention comprise the step of identifying a subject or subject group having one or more of the following: baseline non-HDL-C value of about 200 mg/dL to about 400 mg/dL, for example at least about 210 mg/dL, at least about 220 mg/dL, at least about 230 mg/dL, at least about 240 mg/dL, at least about 250 mg/dL, at least about 260 mg/dL, at least about 270 mg/dL, at least about 280 mg/dL, at least about 290 mg/dL, or at least about 300 mg/dL; baseline total cholesterol value of about 250 mg/dL to about 400 mg/dL, for example at least about 260 mg/dL, at least about 270 mg/dL, at least about 280 mg/dL or at least about 290 mg/dL; baseline VLDL-C value of about 140 mg/dL to about 200 mg/dL, for example at least about 150 mg/dL, at least about 160 mg/dL, at least about 170 mg/dL, at least about 180 mg/dL or at least about 190 mg/dL; baseline HDL-C value of about 10 to about 60 mg/dL, for example not more than about 40 mg/dL, not more than about 35 mg/dL, not more than about 30 mg/dL, not more than about 25 mg/dL, not more than about 20 mg/dL, or not more than about 15 mg/dL; and/or baseline LDL-C value of about 50 to about 300 mg/dL, for example not less than about 100 mg/dL, not less than about 90 mg/dL, not less than about 80 mg/dL, not less than about 70 mg/dL, not less than about 60 mg/dL or not less than about 50 mg/dL.

In one embodiment, a composition of the invention is administered to a subject in an amount sufficient to provide a daily dose of eicosapentaenoic acid of about 1 mg to about 10,000 mg, 25 about 5000 mg, about 50 to about 3000 mg, about 75 mg to about 2500 mg, or about 100 mg to about 1000 mg, for example about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1025 mg, about 1050 mg, about 1075 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125 mg, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg, about 2500 mg, about 2525 mg, about 2550 mg, about 2575 mg, about 2600 mg, about 2625 mg, about 2650 mg, about 2675 mg, about 2700 mg, about 2725 mg, about 2750 mg, about 2775 mg, about 2800 mg, about 2825 mg, about 2850 mg, about 2875 mg, about 2900 mg, about 2925 mg, about 2950 mg, about 2975 mg, about 3000 mg, about 3025 mg, about 3050 mg, about 3075 mg, about 3100 mg, about 3125 mg, about 3150 mg, about 3175 mg, about 3200 mg, about 3225 mg, about 3250 mg, about 3275 mg, about 3300 mg, about 3325 mg, about 3350 mg, about 3375 mg, about 3400 mg, about 3425 mg, about 3450 mg, about 3475 mg, about 3500 mg, about 3525 mg, about 3550 mg, about 3575 mg, about 3600 mg, about 3625 mg, about 3650 mg, about 3675 mg, about 3700 mg, about 3725 mg, about 3750 mg, about 3775 mg, about 3800 mg, about 3825 mg, about 3850 mg, about 3875 mg, about 3900 mg, about 3925 mg, about 3950 mg, about 3975 mg, about 4000 mg, about 4025 mg, about 4050 mg, about 4075 mg, about 4100 mg, about 4125 mg, about 4150 mg, about 4175 mg, about 4200 mg, about 4225 mg, about 4250 mg, about 4275 mg, about 4300 mg, about 4325 mg, about 4350 mg, about 4375 mg, about 4400 mg, about 4425 mg, about 4450 mg, about 4475 mg, about 4500 mg, about 4525 mg, about 4550 mg, about 4575 mg, about 4600 mg, about 4625 mg, about 4650 mg, about 4675 mg, about 4700 mg, about 4725 mg, about 4750 mg, about 4775 mg, about 4800 mg, about 4825 mg, about 4850 mg, about 4875 mg, about 4900 mg, about 4925 mg, about 4950 mg, about 4975 mg, about 5000 mg, about 5025 mg, about 5050 mg, about 5075 mg, about 5100 mg, about 5125 mg, about 5150 mg, about 5175 mg, about 5200 mg, about 5225 mg, about 5250 mg, about 5275 mg, about 5300 mg, about 5325 mg, about 5350 mg, about 5375 mg, about 5400 mg, about 5425 mg, about 5450 mg, about 5475 mg, about 5500 mg, about 5525 mg, about 5550 mg, about 5575 mg, about 5600 mg, about 5625 mg, about 5650 mg, about 5675 mg, about 5700 mg, about 5725 mg, about 5750 mg, about 5775 mg, about 5800 mg, about 5825 mg, about 5850 mg, about 5875 mg, about 5900 mg, about 5925 mg, about 5950 mg, about 5975 mg, about 6000 mg, about 6025 mg, about 6050 mg, about 6075 mg, about 6100 mg, about 6125 mg, about 6150 mg, about 6175 mg, about 6200 mg, about 6225 mg, about 6250 mg, about 6275 mg, about 6300 mg, about 6325 mg, about 6350 mg, about 6375 mg, about 6400 mg, about 6425 mg, about 6450 mg, about 6475 mg, about 6500 mg, about 6525 mg, about 6550 mg, about 6575 mg, about 6600 mg, about 6625 mg, about 6650 mg, about 6675 mg, about 6700 mg, about 6725 mg, about 6750 mg, about 6775 mg, about 6800 mg, about 6825 mg, about 6850 mg, about 6875 mg, about 6900 mg, about 6925 mg, about 6950 mg, about 6975 mg, about 7000 mg, about 7025 mg, about 7050 mg, about 7075 mg, about 7100 mg, about 7125 mg, about 7150 mg, about 7175 mg, about 7200 mg, about 7225 mg, about 7250 mg, about 7275 mg, about 7300 mg, about 7325 mg, about 7350 mg, about 7375 mg, about 7400 mg, about 7425 mg, about 7450 mg, about 7475 mg, about 7500 mg, about 7525 mg, about 7550 mg, about 7575 mg, about 7600 mg, about 7625 mg, about 7650 mg, about 7675 mg, about 7700 mg, about 7725 mg, about 7750 mg, about 7775 mg, about 7800 mg, about 7825 mg, about 7850 mg, about 7875 mg, about 7900 mg, about 7925 mg, about 7950 mg, about 7975 mg, about 8000 mg, about 8025 mg, about 8050 mg, about 8075 mg, about 8100 mg, about 8125 mg, about 8150 mg, about 8175 mg, about 8200 mg, about 8225 mg, about 8250 mg, about 8275 mg, about 8300 mg, about 8325 mg, about 8350 mg, about 8375 mg, about 8400 mg, about 8425 mg, about 8450 mg, about 8475 mg, about 8500 mg, about 8525 mg, about 8550 mg, about 8575 mg, about 8600 mg, about 8625 mg, about 8650 mg, about 8675 mg, about 8700 mg, about 8725 mg, about 8750 mg, about 8775 mg, about 8800 mg, about 8825 mg, about 8850 mg, about 8875 mg, about 8900 mg, about 8925 mg, about 8950 mg, about 8975 mg, about 9000 mg, about 9025 mg, about 9050 mg, about 9075 mg, about 9100 mg, about 9125 mg, about 9150 mg, about 9175 mg, about 9200 mg, about 9225 mg, about 9250 mg, about 9275 mg, about 9300 mg, about 9325 mg, about 9350 mg, about 9375 mg, about 9400 mg, about 9425 mg, about 9450 mg, about 9475 mg, about 9500 mg, about 9525 mg, about 9550 mg, about 9575 mg, about 9600 mg, about 9625 mg, about 9650 mg, about 9675 mg, about 9700 mg, about 9725 mg, about 9750 mg, about 9775 mg, about 9800 mg, about 9825 mg, about 9850 mg, about 9875 mg, about 9900 mg, about 9925 mg, about 9950 mg, about 9975 mg, or about 10,000 mg.

In another embodiment, any of the methods disclosed herein are used in treatment or prevention of a subject or subjects that consume a traditional Western diet. In one embodiment, the methods of the invention include a step of identifying a subject as a Western diet consumer or prudent diet consumer and then treating the subject if the subject is deemed a Western diet consumer. The term "Western diet" herein refers generally to a typical diet consisting of, by percentage of total calories, about 45% to about 50% carbohydrate, about 35% to about 40% fat, and about 10% to about 15% protein. A Western diet may alternately or additionally be characterized by relatively high intakes of red and processed meats, sweets, refined grains, and desserts, for example more than 50%, more than 60% or more or 70% of total calories come from these sources.

In one embodiment, a composition for use in methods of the invention comprises eicosapentaenoic acid, or a pharmaceutically acceptable ester, derivative, conjugate or salt thereof, or mixtures of any of the foregoing, collectively referred to herein as "EPA." The term "pharmaceutically acceptable" in the present context means that the substance in question does not produce unacceptable toxicity to the subject or interaction with other components of the composition.

In one embodiment, the EPA comprises all-cis eicosa-5,8,11,14,17-pentaenoic acid. In another embodiment, the EPA comprises an eicosapentaenoic acid ester. In another embodiment, the EPA comprises a C₁ - C₅ alkyl ester of eicosapentaenoic acid. In another embodiment, the EPA comprises eicosapentaenoic acid ethyl ester, eicosapentaenoic acid methyl ester, eicosapentaenoic acid propyl ester, or eicosapentaenoic acid butyl ester. In another embodiment, the EPA comprises In one embodiment, the EPA comprises all-cis eicosa-5,8,11,14,17-pentaenoic acid ethyl ester.

In another embodiment, the EPA is in the form of ethyl-EPA, lithium EPA, mono-, di- or triglyceride EPA or any other ester or salt of EPA, or the free acid form of EPA. The EPA may also be in the form of a 2-substituted derivative or other derivative which slows down its rate of oxidation but does not otherwise change its biological action to any substantial degree.

In another embodiment, EPA is present in a composition useful in accordance with methods of the invention in an amount of about 50 mg to about 5000 mg, about 75 mg to about 2500 mg, or about 100 mg to about 1000 mg, for example about 50 mg, about 75 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 525 mg, about 550 mg, about 575 mg, about 600 mg, about 625 mg, about 650 mg, about 675 mg, about 700 mg, about 725 mg, about 750 mg, about 775 mg, about 800 mg, about 825 mg, about 850 mg, about 875 mg, about 900 mg, about 925 mg, about 950 mg, about 975 mg, about 1000 mg, about 1025 mg, about 1050 mg, about 1075 mg, about 1100 mg, about 1025 mg, about 1050 mg, about 1075 mg, about 1200 mg, about 1225 mg, about 1250 mg, about 1275 mg, about 1300 mg, about 1325 mg, about 1350 mg, about 1375 mg, about 1400 mg, about 1425 mg, about 1450 mg, about 1475 mg, about 1500 mg, about 1525 mg, about 1550 mg, about 1575 mg, about 1600 mg, about 1625 mg, about 1650 mg, about 1675 mg, about 1700 mg, about 1725 mg, about 1750 mg, about 1775 mg, about 1800 mg, about 1825 mg, about 1850 mg, about 1875 mg, about 1900 mg, about 1925 mg, about 1950 mg, about 1975 mg, about 2000 mg, about 2025 mg, about 2050 mg, about 2075 mg, about 2100 mg, about 2125 mg, about 2150 mg, about 2175 mg, about 2200 mg, about 2225 mg, about 2250 mg, about 2275 mg, about 2300 mg, about 2325 mg, about 2350 mg, about 2375 mg, about 2400 mg, about 2425 mg, about 2450 mg, about 2475 mg, about 2500 mg, about 2525 mg, about 2550 mg, about 2575 mg, about 2600 mg, about 2625 mg, about 2650 mg, about 2675 mg, about 2700 mg, about 2725 mg, about 2750 mg, about 2775 mg, about 2800 mg, about 2825 mg, about 2850 mg, about 2875 mg, about 2900 mg, about 2925 mg, about 2950 mg, about 2975 mg, about 3000 mg, about 3025 mg, about 3050 mg, about 3075 mg, about 3100 mg, about 3125 mg, about 3150 mg, about 3175 mg, about 3200 mg, about 3225 mg, about 3250 mg, about 3275 mg, about 3300 mg, about 3325 mg, about 3350 mg, about 3375 mg, about 3400 mg, about 3425 mg, about 3450 mg, about 3475 mg, about 3500 mg, about 3525 mg, about 3550 mg, about 3575 mg, about 3600 mg, about 3625 mg, about 3650 mg, about 3675 mg, about 3700 mg, about 3725 mg, about 3750 mg, about 3775 mg, about 3800 mg, about 3825 mg, about 3850 mg, about 3875 mg, about 3900 mg, about 3925 mg, about 3950 mg, about 3975 mg, about 4000 mg, about 4025 mg, about 4050 mg, about 4075 mg, about 4100 mg, about 4125 mg, about 4150 mg, about 4175 mg, about 4200 mg, about 4225 mg, about 4250 mg, about 4275 mg, about 4300 mg, about 4325 mg, about 4350 mg, about 4375 mg, about 4400 mg, about 4425 mg, about 4450 mg, about 4475 mg, about 4500 mg, about 4525 mg, about 4550 mg, about 4575 mg, about 4600 mg, about 4625 mg, about 4650 mg, about 4675 mg, about 4700 mg, about 4725 mg, about 4750 mg, about 4775 mg, about 4800 mg, about 4825 mg, about 4850 mg, about 4875 mg, about 4900 mg, about 4925 mg, about 4950 mg, about 4975 mg, or about 5000 mg.

In another embodiment, a composition useful in accordance with the invention contains not more than about 10%, not more than about 9%, not more than about 8%, not more than about 7%, not more than about 6%, not more than about 5%, not more than about 4%, not more than about 3%, not more than about 2%, not more than about 1%, or not more than about 0.5%, by weight, docosahexaenoic acid (DHA), if any. In another embodiment, a composition of the invention contains substantially no docosahexaenoic acid. In still another embodiment, a composition useful in the present invention contains no docosahexaenoic acid and/or derivative thereof.

In another embodiment, EPA comprises at least 70%, at least 80%, at least 90%, at least 95%, at least 96%, at least 97%, at least 98%, at least 99%, or 100%, by weight, of all fatty acids present in a composition that is useful in methods of the present invention.

In one embodiment, a composition of the invention comprises ultra-pure EPA. The term "ultra-pure" as used herein with respect to EPA refers to a composition comprising at least 95% by weight EPA (as the term "EPA" is defined and exemplified herein). Ultra-pure EPA comprises at least 96% by weight EPA, at least 97% by weight EPA, or at least 98% by weight EPA, wherein the EPA is any form of EPA as set forth herein.

In another embodiment, a composition useful in accordance with methods of the invention contains less than 10%, less than 9%, less than 8%, less than 7%, less than 6%, less than 5%, less than 4%, less than 3%, less than 2%, less than 1%, less than 0.5% or less than 0.25%, by weight of the total composition or by weight of the total fatty acid content, of any fatty acid other than EPA. Illustrative examples of a "fatty acid other than EPA" include linolenic acid (LA), arachidonic acid (AA), docosahexaenoic acid (DHA), alpha-linolenic acid (ALA), stearadonic acid (STA), eicosatrienoic acid (ETA) and/or docosapentaenoic acid (DPA). In another embodiment, a composition useful in accordance with methods of the invention contains about 0.1% to about 4%, about 0.5% to about 3%, or about 1% to about 2%, by weight, of total fatty acids other than EPA and/or DHA.

In another embodiment, a composition useful in accordance with the invention has one or more of the following features: (a) eicosapentaenoic acid ethyl ester represents at least about 96%, at least about 97%, or at least about 98%, by weight, of all fatty acids present in the composition; (b) the composition contains not more than about 4%, not more than about 3%, or not more than about 2%, by weight, of total fatty acids other than eicosapentaenoic acid ethyl ester; (c) the composition contains not more than about 0.6%, not more than about 0.5%, or not more than about 0.4% of any individual fatty acid other than eicosapentaenoic acid ethyl ester; (d) the composition has a refractive index (20 °C) of about 1 to about 2, about 1.2 to about 1.8 or about 1.4 to about 1.5; (e) the composition has a specific gravity (20 °C) of about 0.8 to about 1.0, about 0.85 to about 0.95 or about 0.9 to about 0.92; (e) the composition contains not more than about 20 ppm, not more than about 15 ppm or not more than about 10 ppm heavy metals, (f) the composition contains not more than about 5 ppm, not more than about 4 ppm, not more than about 3 ppm, or not more than about 2 ppm arsenic, and/or (g) the composition has a peroxide value of not more than about 5 meq/kg, not more than about 4 meq/kg, not more than about 3 meq/kg, or not more than about 2 meq/kg.

In another embodiment, a composition useful in accordance with the invention comprises, consists of or consists essentially of at least 95% by weight ethyl eicosapentaenoate (EPA-E), about 0.2% to about 0.5% by weight ethyl octadecatetraenoate (ODTA-E), about 0.05% to about 0.25% by weight ethyl nonaecapentaenoate (NDPA-E), about 0.2% to about 0.45% by weight ethyl arachidonate (AA-E), about 0.3% to about 0.5% by weight ethyl eicosatetraenoate (ETA-E), and about 0.05% to about 0.32% ethyl heneicosapentaenoate (HPA-E). In another embodiment, the composition is present in a capsule shell.

In another embodiment, compositions useful in accordance with the invention comprise, consist essential of, or consist of at least 95%, 96% or 97%, by weight, ethyl eicosapentaenoate, about 0.2% to about 0.5% by weight ethyl octadecatetraenoate, about 0.05% to about 0.25% by weight ethyl nonaecapentaenoate, about 0.2% to about 0.45% by weight ethyl arachidonate, about 0.3% to about 0.5% by weight ethyl eicosatetraenoate, and about 0.05% to about 0.32% ethyl heneicosapentaenoate. Optionally, the composition contains not more than about 0.06%, about 0.05%, or about 0.04%, by weight, DHA or derivative thereof such as ethyl-DHA. In one embodiment the composition contains substantially no or no amount of DHA or derivative thereof such as ethyl-DHA. The composition further optionally comprises one or more antioxidants (e.g. tocopherol) or other impurities in an amount of not more than about 0.5% or not more than 0.05%. In another embodiment, the composition comprises about 0.05% to about 0.4%, for example about 0.2% by weight tocopherol. In another embodiment, about 500 mg to about 1 g of the composition is provided in a capsule shell.

In another embodiment, compositions useful in accordance with the invention comprise, consist essential of, or consist of at least 96% by weight ethyl eicosapentaenoate, about 0.22% to about 0.4% by weight ethyl octadecatetraenoate, about 0.075% to about 0.20% by weight ethyl nonaecapentaenoate, about 0.25% to about 0.40% by weight ethyl arachidonate, about 0.3% to about 0.4% by weight ethyl eicosatetraenoate and about 0.075% to about 0.25% ethyl heneicosapentaenoate. Optionally, the composition contains not more than about 0.06%, about 0.05%, or about 0.04%, by weight, DHA or derivative thereof such as ethyl-DHA. In one embodiment the composition contains substantially no or no amount of DHA or derivative thereof such as ethyl-DHA. The composition further optionally comprises one or more antioxidants (e.g. tocopherol) or other impurities in an amount of not more than about 0.5% or not more than 0.05%. In another embodiment, the composition comprises about 0.05% to about 0.4%, for example about 0.2% by weight tocopherol. In another embodiment, the invention provides a dosage form comprising about 500 mg to about 1 g of the foregoing composition in a capsule shell. In one embodiment, the dosage form is a gel or liquid capsule and is packaged in blister packages of about 1 to about 20 capsules per sheet.

In another embodiment, compositions useful in accordance with the invention comprise, consist essential of, or consist of at least 96%, 97% or 98%, by weight, ethyl eicosapentaenoate, about 0.25% to about 0.38% by weight ethyl octadecatetraenoate, about 0.10% to about 0.15% by weight ethyl nonaecapentaenoate, about 0.25% to about 0.35% by weight ethyl arachidonate, about 0.31% to about 0.38% by weight ethyl eicosatetraenoate, and about 0.08% to about 0.20% ethyl heneicosapentaenoate. Optionally, the composition contains not more than about 0.06%, about 0.05%, or about 0.04%, by weight, DHA or derivative thereof such as ethyl-DHA. In one embodiment the composition contains substantially no or no amount of DHA or derivative thereof such as ethyl-DHA. The composition further optionally comprises one or more antioxidants (e.g. tocopherol) or other impurities in an amount of not more than about 0.5% or not more than 0.05%. In another embodiment, the composition comprises about 0.05% to about 0.4%, for example about 0.2% by weight tocopherol. In another embodiment, the invention provides a dosage form comprising about 500 mg to about 1 g of the foregoing composition in a capsule shell.

In another embodiment, a composition as described herein is administered to a subject once or twice per day. In another embodiment, 1, 2, 3 or 4 capsules, each containing about 1 g of a composition as described herein, are administered to a subject daily. In another embodiment, 1 or 2 capsules, each containing about 1 g of a composition as described herein, are administered to the subject in the morning, for example between about 5 am and about 11 am, and 1 or 2 capsules, each containing about 1 g of a composition as described herein, are administered to the subject in the evening, for example between about 5 pm and about 11 pm.

In one embodiment, a subject being treated in accordance with methods of the invention is not otherwise on lipid-altering therapy, for example statin, fibrate, niacin and/or ezetimibe therapy.

In another embodiment, compositions useful in accordance with methods of the invention are orally deliverable. The terms "orally deliverable" or "oral administration" herein include any form of delivery of a therapeutic agent or a composition thereof to a subject wherein the agent or composition is placed in the mouth of the subject, whether or not the agent or composition is swallowed. Thus "oral administration" includes buccal and sublingual as well as esophageal administration. In one embodiment, the composition is present in a capsule, for example a soft gelatin capsule.

A composition for use in accordance with the invention can be formulated as one or more dosage units. The terms "dose unit" and "dosage unit" herein refer to a portion of a pharmaceutical composition that contains an amount of a therapeutic agent suitable for a single administration to provide a therapeutic effect. Such dosage units may be administered one to a plurality (*i.e.* 1 to about 10, 1 to 8, 1 to 6, 1 to 4 or 1 to 2) of times per day, or as many times as needed to elicit a therapeutic response.

In another embodiment, the invention provides use of any composition described herein for treating moderate to severe hypertriglyceridemia in a subject in need thereof, comprising: providing a subject having a fasting baseline triglyceride level of about 500 mg/dl to about 1500 mg/dl and administering to the subject a pharmaceutical composition as described herein. In one embodiment, the composition comprises about 1 g to about 4 g of eicosapentaenoic acid ethyl ester, wherein the composition contains substantially no docosahexaenoic acid.

In one embodiment, compositions of the invention, upon storage in a closed container maintained at room temperature, refrigerated (*e.g.* about 5 to about 5 -10 °C) temperature, or frozen for a period of about 1, 2, 3, 4, 5, 6, 7, 8, 9, 10, 11, or 12 months, exhibit at least about 90%, at least about 95%, at least about 97.5%, or at least about 99% of the active ingredient(s) originally present therein.

In one embodiment, the invention provides use of a composition as described herein in manufacture of a medicament for treatment of any of a cardiovascular-related disease. In another embodiment, the subject is diabetic.

In one embodiment, a composition as set forth herein is packaged together with instructions for using the composition to treat pediatric metabolic syndrome.

Aspects of the invention are defined in the following numbered paragraphs:
1. A method of treating a pediatric metabolic syndrome in a subject in need thereof, the method comprising:
   (a) identifying a subject as having pediatric metabolic syndrome; and
   (b) administering to the subject about 1 g to about 4 g of ethyl eicosapentaenoate per day.
2. The method of numbered paragraph 1, wherein the subject has one or more of:
   excessive belly fat, hypertension, hyperlipidemia, and/or hyperglycemia.
3. The method of numbered paragraph 1 or numbered paragraph 2, wherein the subject has three or more of: (a) a fasting glucose level of at least 110 mg/dL; (b) a waist circumference value in at least the 90th percentile based on an age and a gender associated with the subject; (c) a triglyceride level of at least 110 mg/dL; (d) an HDL-C level of no more than about 40 mg/dL; and (e) a blood pressure level in at least the 90th percentile based on the age, gender and height associated with the subject.
4. The method of any one preceding numbered paragraph, wherein the subject has three or more of: (a) a fasting glucose level of at least 110 mg/dL; (b) a waist circumference value in at least the 75th percentile; (c) a triglyceride level of at least 100 mg/dL; (d) an HDL-C level of no more than about 50 mg/dL; and (e) a blood pressure level in at least the 90th percentile.
5. The method of any one preceding numbered paragraph, wherein the subject has three or more of: (a) an impaired glucose tolerance as determined by having two-hour glucose levels of 140 mg/dL to 199 mg/dL on the 75-gram oral glucose tolerance test; (b) a waist circumference value in at least the 90th percentile based on an age, a gender, and a race associated with the subject; (c) a triglyceride level in at least the 90th percentile based on the age and gender associated with the subject; (d) an HDL-C level in no more than the 10th percentile based on the age and gender associated with the subject; and (e) a blood pressure level in at least the 90th percentile based on the age, the gender and a height associated with the subject.
6. The method of any one preceding numbered paragraph, wherein the subject has three or more of: (a) an impaired glucose tolerance as determined by having two-hour glucose levels of 140 mg/dL to 199 mg/dL on the 75-gram oral glucose tolerance test; (b) a body mass index ("BMI-Z") score of at least 2.0 based on an age and a gender associated with the subject; (c) a triglyceride level in at least the 95th percentile based on the age, the gender and a race associated with the subject; (d) an HDL-C level in no more than the 5th percentile based on the age, the gender, and the race associated with the subject; and (e) a blood pressure level in at least the 95th percentile based on the age, gender and height associated with the subject.
7. The method of any one preceding numbered paragraph, wherein the subject has three or more of: (a) a fasting glucose level of at least 110 mg/dL; (b) a waist circumference value in at least the 90th percentile based on a gender associated with the subject; (c) a triglyceride level of at least 110 mg/dL based on the age associated with the subject; (d) an HDL-C level of no more than 40 mg/dL; and (e) a blood pressure level in at least the 90th percentile based on the age, the gender and a height associated with the subject.
8. The method of any one preceding numbered paragraph, wherein the subject has an age of at least 6 years and less than 10 years and has a waist circumference value in at least the 90th percentile.
9. The method of any one preceding numbered paragraph, wherein the subject has an age of at least 10 years and less than 16 years and a waist circumference value in at least the 90th percentile; and further has at least two of: (a) a triglyceride level of at least 150 mg/dL; (b) an HDL-C level of no more than 40 mg/dL; (c) a blood pressure of at least 130 mmHg systolic or at least 85 mmHg diastolic; and (d) a blood glucose level of at least 100 mg/dL.
10. The method of any one preceding numbered paragraph, wherein the subject:
   has an age of at least 16 years;
   has a body mass index of at least 30 kg/m² or a waist measurement of:
      at least 102 cm if the subject is male and lives in North America or at least 88 cm if the subject is female and lives in North America,
      at least 94 cm if the subject is male, does not live in North America, and has an ethnicity associated with Europe, sub-Saharan Africa, Eastern Mediterranean or the Middle East, or at least 80 cm if the subject is female, does not live in North America, and has an ethnicity associated with Europe, sub-Sȧharan Africa, Eastern Mediterranean or the Middle East, or
      at least 90 cm if the subject is male, does not live in North America, and has an ethnicity associated with South Asia, China, Japan, South America or Central America, or at least 80 cm if the subject is female, does not live in North America, and has an ethnicity associated with South Asia, China, Japan, South America or Central America; and
   has at least two of: (a) a triglyceride level of at least 150 mg/dL; (b) an HDL-C level of no more than 40 mg/dL if the subject is male or of no more than 50 mg/dL if the subject is female; (c) a blood pressure of at least 130 mmHg systolic or at least 85 mmHg diastolic; and (d) a fasting plasma glucose level of at least 100 mg/dL.
11. The method of any one preceding numbered paragraph, wherein the ethyl eicosapentaenoate represents at least about 80%, by weight, of all fatty acids administered to the subject.
12. The method of any one preceding numbered paragraph, wherein the ethyl eicosapentaenoate represents at least about 90%, by weight, of all fatty acids administered to the subject.
13. The method of any one preceding numbered paragraph, wherein the ethyl eicosapentaenoate represents at least about 95%, by weight, of all fatty acids administered to the subject.
14. The method of any one preceding numbered paragraph, wherein docosahexaenoic acid and its derivatives represent less than about 10%, by weight, of all fatty acids administered to the subject.
15. The method of any one preceding numbered paragraph, wherein docosahexaenoic acid and its derivatives represent less than about 5%, by weight, of all fatty acids administered to the subject.
16. The method of numbered paragraph 1, wherein the subject is not on concomitant lipid-altering therapy.
17. The method of any one preceding numbered paragraph, further comprising a step of measuring a baseline lipid profile in the subject prior to administering the pharmaceutical composition to said subject.
18. The method of numbered paragraph 17, wherein the subject has one or more of: a fasting baseline triglyceride level of about 135 mg/dL to about 1500 mg/dL, a baseline non-HDL-C value of about 200 mg/dL to about 300 mg/dL; a baseline total cholesterol value of about 250 mg/dL to about 300 mg/dL; a baseline VLDL-C value of about 140 mg/dL to about 200 mg/dL; and/or a baseline HDL-C value of about 10 to about 80 mg/dL.
19. The method of numbered paragraph 17 or numbered paragraph 18 wherein after administering to the subject said pharmaceutical composition daily for about 12 weeks, the subject exhibits one or more of: (a) reduced triglyceride levels compared to baseline; (b) reduced Apo B levels compared to baseline; (c) increased HDL-C levels compared to baseline; (d) a reduction in non-HDL-C levels compared to baseline; and/or (e) a reduction in VLDL levels compared to baseline.
20. The method of numbered paragraph 19 wherein the subject exhibits one or more of: (a) a reduction in triglyceride level of at least about 5% as compared to baseline; (b) a less than 30% increase in non-HDL-C levels or a reduction in non-HDL-C levels of at least about 1% as compared to baseline; (c) an increase in HDL-C levels of at least about 5% as compared to baseline; and/or (d) a less than 60% increase in LDL-C levels compared to baseline.
21. The method of numbered paragraph 19 wherein the subject exhibits one or more of: (a) a reduction in triglyceride level of at least about 30% as compared to baseline; (b) no increase in non-HDL-C levels as compared to baseline; (c) no decrease in HDL-C levels compared to baseline; and/or (d) a less than 30% increase in LDL-C levels as compared to baseline.
22. The method of any one preceding numbered paragraph, wherein upon treatment the subject exhibits one or more of the following outcomes: (a) reduced triglyceride levels compared to baseline; (b) reduced Apo B levels compared to baseline; (c) increased HDL-C levels compared to baseline; (d) no increase in LDL-C levels compared to baseline; (e) a reduction in LDL-C levels compared to baseline; (f) a reduction in non-HDL-C levels compared to baseline; (g) a reduction in VLDL levels compared to baseline; (h) an increase in apo A-I levels compared to baseline; (i) an increase in apo A-I/apo B ratio compared to baseline; (j) a reduction in lipoprotein a levels compared to baseline; (k) a reduction in LDL particle number compared to baseline; (1) an increase in LDL size compared to baseline; (m) a reduction in remnant-like particle cholesterol compared to baseline; (n) a reduction in oxidized LDL compared to baseline; (o) a less than 5% change in fasting plasma glucose (FPG) compared to baseline; (p) a less than 5% change in hemoglobin A_{1c} (HbA_{1c}) compared to baseline; (q) a reduction in homeostasis model insulin resistance compared to baseline; (r) a reduction in lipoprotein associated phospholipase A2 compared to baseline; (s) a reduction in intracellular adhesion molecule compared to baseline; (t) a reduction in interleukin-6 compared to baseline; (u) a reduction in plasminogen activator inhibitor compared to baseline; (v) a reduction in high sensitivity C-reactive protein (hsCRP) compared to baseline; (w) an increase in serum phospholipid EPA compared to baseline; and/or (x) an increase in red blood cell membrane EPA compared to baseline.
23. The method of any one preceding numbered paragraph, wherein the subject is pre-diabetic.
24. The method of any one preceding numbered paragraph, wherein the ethyl eicosapentaenoate is packaged together with instructions for using the composition to treat pediatric metabolic syndrome.

## Claims

1. A pharmaceutical composition for use in treating pediatric metabolic syndrome in a subject in need thereof, wherein the composition comprises ethyl eicosapetaenoate and about 1 g to about 4 g of ethyl eicosapentaenoate is administered to the subject per day.

2. The composition for use for the use according to claim 1, wherein the subject has one or more of: excessive belly fat, hypertension, hyperlipidemia, and/or hyperglycemia.

3. The composition for the use according to claim 1 or claim 2, wherein:
the subject has three or more of: (a) a fasting glucose level of at least 110 mg/dL; (b) a waist circumference value in at least the 90th percentile based on an age and a gender associated with the subject; (c) a triglyceride level of at least 110 mg/dL; (d) an HDL-C level of no more than about 40 mg/dL; and (e) a blood pressure level in at least the 90th percentile based on the age, gender and height associated with the subject; and/or
the subject has three or more of: (a) a fasting glucose level of at least 110 mg/dL; (b) a waist circumference value in at least the 75th percentile; (c) a triglyceride level of at least 100 mg/dL; (d) an HDL-C level of no more than about 50 mg/dL; and (e) a blood pressure level in at least the 90th percentile; and/or
the subject has three or more of: (a) an impaired glucose tolerance as determined by having two-hour glucose levels of 140 mg/dL to 199 mg/dL on the 75-gram oral glucose tolerance test; (b) a waist circumference value in at least the 90th percentile based on an age, a gender, and a race associated with the subject; (c) a triglyceride level in at least the 90th percentile based on the age and gender associated with the subject; (d) an HDL-C level in no more than the 10th percentile based on the age and gender associated with the subject; and (e) a blood pressure level in at least the 90th percentile based on the age, the gender and a height associated with the subject.

4. The composition for the use according to any preceding claim, wherein
the subject has three or more of: (a) an impaired glucose tolerance as determined by having two-hour glucose levels of 140 mg/dL to 199 mg/dL on the 75-gram oral glucose tolerance test; (b) a body mass index ("BMI-Z") score of at least 2.0 based on an age and a gender associated with the subject; (c) a triglyceride level in at least the 95th percentile based on the age, the gender and a race associated with the subject; (d) an HDL-C level in no more than the 5th percentile based on the age, the gender, and the race associated with the subject; and (e) a blood pressure level in at least the 95th percentile based on the age, gender and height associated with the subject; and/or
the subject has three or more of: (a) a fasting glucose level of at least 110 mg/dL; (b) a waist circumference value in at least the 90th percentile based on a gender associated with the subject; (c) a triglyceride level of at least 110 mg/dL based on the age associated with the subject; (d) an HDL-C level of no more than 40 mg/dL; and (e) a blood pressure level in at least the 90th percentile based on the age, the gender and a height associated with the subject.

5. The composition for the use according to any preceding claim, wherein
the subject has an age of at least 6 years and less than 10 years and has a waist circumference value in at least the 90th percentile; and/or
the subject has an age of at least 10 years and less than 16 years and a waist circumference value in at least the 90th percentile; and further has at least two of: (a) a triglyceride level of at least 150 mg/dL; (b) an HDL-C level of no more than 40 mg/dL; (c) a blood pressure of at least 130 mmHg systolic or at least 85 mmHg diastolic; and (d) a blood glucose level of at least 100 mg/dL.

6. The composition for the use according to any preceding claim, wherein the subject:
has an age of at least 16 years;
has a body mass index of at least 30 kg/m² or a waist measurement of:
at least 102 cm if the subject is male and lives in North America or at least 88 cm if the subject is female and lives in North America,
at least 94 cm if the subject is male, does not live in North America, and has an ethnicity associated with Europe, sub-Saharan Africa, Eastern Mediterranean or the Middle East, or at least 80 cm if the subject is female, does not live in North America, and has an ethnicity associated with Europe, sub-Saharan Africa, Eastern Mediterranean or the Middle East, or
at least 90 cm if the subject is male, does not live in North America, and has an ethnicity associated with South Asia, China, Japan, South America or Central America, or at least 80 cm if the subject is female, does not live in North America, and has an ethnicity associated with South Asia, China, Japan, South America or Central America; and
has at least two of: (a) a triglyceride level of at least 150 mg/dL; (b) an HDL-C level of no more than 40 mg/dL if the subject is male or of no more than 50 mg/dL if the subject is female; (c) a blood pressure of at least 130 mmHg systolic or at least 85 mmHg diastolic; and (d) a fasting plasma glucose level of at least 100 mg/dL.

7. The composition for the use according to any preceding claim, wherein the ethyl eicosapentaenoate represents at least about 80%, by weight, of all fatty acids in the composition,
optionally wherein the ethyl eicosapentaenoate represents at least about 90%, by weight, of all fatty acids in the composition, and
optionally wherein the ethyl eicosapentaenoate represents at least about 95%, by weight, of all fatty acids in the composition.

8. The composition for the use according to any preceding claim, wherein further wherein docosahexaenoic acid and its derivatives represent less than about 10%, by weight, of all fatty acids in the composition,
optionally wherein docosahexaenoic acid and its derivatives represent less than about 5%, by weight, of all fatty acids in the composition.

9. The composition for the use according to claim 1, wherein the subject is not on concomitant lipid-altering therapy; and/or wherein the subject's baseline lipid profile is measured prior to administering the said medicament.

10. The composition for the use according to claim 9, wherein the subject has one or more of: a fasting baseline triglyceride level of about 135 mg/dL to about 1500 mg/dL, a baseline non-HDL-C value of about 200 mg/dL to about 300 mg/dL; a baseline total cholesterol value of about 250 mg/dL to about 300 mg/dL; a baseline VLDL-C value of about 140 mg/dL to about 200 mg/dL; and/or a baseline HDL-C value of about 10 to about 80 mg/dL.

11. The composition for the use according to claim 9 or claim 10 wherein after administering to the subject said medicament daily for about 12 weeks, the subject exhibits one or more of: (a) reduced triglyceride levels compared to baseline; (b) reduced Apo B levels compared to baseline; (c) increased HDL-C levels compared to baseline; (d) a reduction in non-HDL-C levels compared to baseline; and/or (e) a reduction in VLDL levels compared to baseline.

12. The composition for the use according to claim 11 wherein
the subject exhibits one or more of: (a) a reduction in triglyceride level of at least about 5% as compared to baseline; (b) a less than 30% increase in non-HDL-C levels or a reduction in non-HDL-C levels of at least about 1% as compared to baseline; (c) an increase in HDL-C levels of at least about 5% as compared to baseline; and/or (d) a less than 60% increase in LDL-C levels compared to baseline; and/or
the subject exhibits one or more of: (a) a reduction in triglyceride level of at least about 30% as compared to baseline; (b) no increase in non-HDL-C levels as compared to baseline; (c) no decrease in HDL-C levels compared to baseline; and/or (d) a less than 30% increase in LDL-C levels as compared to baseline.

13. The composition for the use according to any preceding claim, wherein upon treatment the subject exhibits one or more of the following outcomes: (a) reduced triglyceride levels compared to baseline; (b) reduced Apo B levels compared to baseline; (c) increased HDL-C levels compared to baseline; (d) no increase in LDL-C levels compared to baseline; (e) a reduction in LDL-C levels compared to baseline; (f) a reduction in non-HDL-C levels compared to baseline; (g) a reduction in VLDL levels compared to baseline; (h) an increase in apo A-I levels compared to baseline; (i) an increase in apo A-I/apo B ratio compared to baseline; (j) a reduction in lipoprotein a levels compared to baseline; (k) a reduction in LDL particle number compared to baseline; (1) an increase in LDL size compared to baseline; (m) a reduction in remnant-like particle cholesterol compared to baseline; (n) a reduction in oxidized LDL compared to baseline; (o) a less than 5% change in fasting plasma glucose (FPG) compared to baseline; (p) a less than 5% change in hemoglobin A1c (HbA1c) compared to baseline; (q) a reduction in homeostasis model insulin resistance compared to baseline; (r) a reduction in lipoprotein associated phospholipase A2 compared to baseline; (s) a reduction in intracellular adhesion molecule compared to baseline; (t) a reduction in interleukin-6 compared to baseline; (u) a reduction in plasminogen activator inhibitor compared to baseline; (v) a reduction in high sensitivity C-reactive protein (hsCRP) compared to baseline; (w) an increase in serum phospholipid EPA compared to baseline; and/or (x) an increase in red blood cell membrane EPA compared to baseline.

14. The composition for the use according to any preceding claim, wherein the subject is pre-diabetic.

15. A composition for use in treating moderate to severe hypertriglyceridemia in a subject having a fasting baseline triglyceride level of about 500 mg/dl to about 1500 mg/dl, the composition comprising about 1 g to about 4 g of ethyl eicosapentaenoate.
